# EUROPÄISCHE PATENTSCHRIFT

(11) **EP 2 453 796 B1**
(45) Veröffentlichungstag und Bekanntmachung des Hinweises auf die Patenterteilung: **19.08.2020**
(21) Anmeldenummer: 10735236.1
(22) Anmeldetag: 14.07.2010
(51) Int. Cl.: A61B 5/151

(54) **OPTIMIERTES LANZETTENBAND**
OPTIMISED LANCET STRIP
BANDE DE LANCETTES OPTIMISÉE

(30) Priorität: 14.07.2009 EP 09165424
(43) Veröffentlichungstag der Anmeldung: 23.05.2012
(73) Patentinhaber: Roche Diabetes Care GmbH, 68305 Mannheim (DE); F. Hoffmann-La Roche AG, 4070 Basel (CH)
(72) Erfinder: KONYA, Ahmet, 68167 Mannheim (DE); HILLER, Bernd, 68623 Lampertheim (DE)
(74) Vertreter: Altmann Stößel Dick Patentanwälte PartG mbB
(86) Internationale Anmeldenummer: PCT/EP2010/060109
(87) Internationale Veröffentlichungsnummer: WO 2011/006913

(56) Entgegenhaltungen:
- EP-A- 1 360 935
- EP-A1- 1 992 284
- WO-A-2007/077212
- WO-A1-2008/138443
- DE-B1- 2 803 345
- US-A1- 2009 010 802

## Beschreibung

### Gebiet der Erfindung

Die Erfindung betrifft ein Verfahren zur Herstellung eines analytischen Bands. Weiterhin betrifft die Erfindung ein analytisches Band sowie ein analytisches Testgerät, welches ein derartiges analytisches Band umfasst. Weiterhin wird eine Vorrichtung zur Herstellung eines analytischen Bands vorgeschlagen. Derartige Verfahren, analytische Bänder, analytische Testgeräte und Vorrichtungen werden insbesondere in der medizinischen Diagnostik, beispielsweise im privaten Bereich, im Pflegebereich oder im Krankenhausbereich eingesetzt, um eine Konzentration eines oder mehrerer Analyte in einer Körperflüssigkeit eines Benutzers zu überwachen, beispielsweise zur Überwachung einer Blutglukosekonzentration. Alternativ oder zusätzlich sind auch andere Anwendungen möglich.

### Stand der Technik

Zur Überwachung einer analytischen Konzentration in einer Körperflüssigkeit eines Benutzers, beispielsweise eines Patienten oder einer für bestimmte Krankheiten anfälligen Person, sind verschiedene Testverfahren bekannt. So sind Testverfahren bekannt, bei welchen der Benutzer beispielsweise bis zu siebenmal täglich eine Probe der Körperflüssigkeit generieren und auf ihre Analytkonzentration untersuchen muss. Um den Tagesablauf des Benutzers durch diesen Zwang zur Untersuchung seiner Körperflüssigkeit, beispielsweise Blut, nicht mehr als nötig zu beeinträchtigen wurden analytische Testgeräte entwickelt, welche einfach und schnell zu handhaben sind und welche beispielsweise als Handgeräte ausgestaltet sind.

Mittlerweile sind analytische Testgeräte bekannt, welche mit einem oder mehreren analytischen Bändern arbeiten, auf welchen mehrere analytische Hilfsmittel aufgebracht sind, die nacheinander verwendet werden können. Diese analytischen Hilfsmittel können beispielsweise Testfelder umfassen, mittels deren eine Probe der Körperflüssigkeit untersucht werden kann. Alternativ oder zusätzlich können die analytischen Hilfsmittel auch Lanzettenelemente umfassen, mittels derer ein Einstich oder Einschnitt in einer Haut des Benutzers vorgenommen werden kann, um auf diese Weise eine Probe der Körperflüssigkeit zu generieren. Die vorliegende Erfindung nimmt insbesondere Bezug auf analytische Bänder mit mehreren Lanzettenelementen, sowie deren Herstellung.

Eine besondere Schwierigkeit bei derartigen analytischen Bändern mit Lanzettenelementen, welche im Folgenden auch als Lanzettenbänder bezeichnet werden, besteht in einer fehlerfreien und sterilen Herstellung dieser Testbänder. Die analytischen Testgeräte weisen in der Regel einen Transportmechanismus auf, beispielsweise einen Transportmechanismus mit einem Greifer, mittels dessen jeweils ein Lanzettenelement in einer Anwendungsposition positionierbar ist. Um den Betriebsablauf der analytischen Testgeräte nicht zu stören, sind dabei hohe Präzisionsanforderungen an die analytischen Bänder zu stellen.

WO 2007/147494 A2 beschreibt ein System, welches medizinische Einweg-Elemente und einen Packstreifen umfasst. Der Packstreifen umfasst eine Mehrzahl von Kammern, in welchen die medizinischen Einweg-Elemente aufgenommen sind. Der Streifen wird gefaltet in dem System aufgenommen.

WO 2008/043565 A2 beschreibt ein Probennahmesystem mit Lanzetten, bei dem automatisch eine Schutzhülle von einer Lanzette entfernt wird und ein Testfeld entpackt wird, bevor diese verwendet werden. Dabei werden Verbindungs-Klebebänder eingesetzt, um die Lanzetten auf ein Trägerband aufzubringen.

Aus EP 1992284 A1 ist ein Verfahren zur Magazinierung von Stechelementen sowie ein Bandmagazin bekannt. Dabei wird eine Vielzahl von mit einer Lanzettenspitze zum Einstechen in die Haut versehenen Einweg-Lanzetten auf einer Spule aufgewickelten oder einem aufwickelbaren Trägerband zum Gewinnen einer Körperflüssigkeitsprobe bereitgestellt. Die Lanzetten werden jeweils in einer Packung angeordnet, wobei die so gebildeten Lanzetten-Packungen einzeln auf das Trägerband aufgebracht und darauf fixiert werden.

Bei bekannten Testbändern besteht jedoch eine technische Herausforderung darin, dass diese Testbänder in analytischen Testgeräten, welche nach einem Weitertakt-Prinzip funktionieren, wie beispielsweise dem in WO 2008/138443 A1 beschriebenen analytischen System, in der Praxis Schwierigkeiten hinsichtlich des Transports bereiten können. So weist ein Weitertakt-Mechanismus in der Regel ein Höhen-empfindliches Element auf, welches auf die Dicke des analytischen Bandes reagiert. Dieses Gerät wird zu Positionierung der Elemente, zum Beispiel der Lanzetten, eingesetzt. Dieser mechanische Sensor reagiert jedoch bei jeder Dickenänderung des analytischen Bandes. Dies kann bei den aus dem Stand der Technik bekannten Bändern somit auch zu nicht gewünschten Zeitpunkten erfolgen, also beispielsweise an einer Kante eines Etiketts in den analytischen Bändern gemäß EP 1992284 A1 oder an einer Faltkante, beispielsweise in dem in WO 2008/043565 A2 beschriebenen analytischen Band.

Ein weiteres dem Stand der Technik entsprechendes System ist z.B. in DE2803345B1 beschrieben.

### Aufgabe der Erfindung

Es ist daher eine Aufgabe der vorliegenden Erfindung, ein analytisches Band, ein Verfahren zu dessen Herstellung sowie ein analytisches Testgerät bereitzustellen, welche die Nachteile des Standes der Technik vermeiden. Insbesondere soll ein Herstellungsverfahren angegeben werden, welches zu analytischen Bändern gleichmäßiger Dicke mit Ausnahme der Erhebung durch die Lanzetten führt, so dass auch ein Weitertakt-Mechanismus sicher und zuverlässig Lanzetten erkennt und diese positionieren kann.

### Offenbarung der Erfindung

Diese Aufgabe wird durch ein Verfahren, ein analytisches Band und ein analytisches Testgerät mit den Merkmalen der unabhängigen Ansprüche gelöst. Vorteilhafte Weiterbildungen der Erfindungen, welche einzeln oder in beliebiger in Kombination realisierbar sind, sind in den abhängigen Ansprüchen dargestellt.

Es wird ein Verfahren zur Herstellung eines analytischen Bandes vorgeschlagen. Bei dem analytischen Band kann es sich insbesondere um ein Lanzettenband handeln, also ein analytisches Band, welches eine Mehrzahl von analytischen Hilfsmitteln in Form von Lanzettenelementen umfasst. Alternativ können, zusätzlich zu den Lanzettenelementen, jedoch auch andere Arten analytischer Hilfsmittel vorgesehen sein, beispielsweise Testfelder, welche mindestens eine Testchemie zum Nachweis mindestens eines Analyten in einer Körperflüssigkeit aufweisen, beispielsweise für einen optischen und/oder elektrochemischen Nachweis des Analyten.

Unter einem Lanzettenelement wird dabei ein grundsätzlich beliebiges Element verstanden, mittels dessen ein Einstich und/oder ein Einschnitt in einer Haut eines Benutzers zum Zwecke einer Generierung einer Probe von Körperflüssigkeit, beispielsweise Blut oder interstitieller Flüssigkeit, generierbar ist. Ein Lanzettenelement kann dementsprechend beispielsweise eine runde oder scharfe Spitze und/oder eine Schneide umfassen. Ein Lanzettenelement kann beispielsweise ganz oder teilweise in Form einer runden Nadel ausgestaltet sein. Besonders bevorzugt ist jedoch, wie unten noch näher ausgeführt wird, die Verwendung von Flachlanzetten, also Lanzetten, welche zwei gegenüberliegende Breitseiten aufweisen und bei denen eine laterale Ausdehnung senkrecht zu einer Einstichrichtung eine Dicke der Lanzetten erheblich übersteigt, beispielsweise um einen Faktor 10 oder mehr. Derartige Flachlanzetten können beispielsweise aus einem Metallband gefertigt sein, beispielsweise durch einen Ätzprozess und/oder einen Stanzprozess und/oder einem Schneideprozess. Zusätzlich zu der Schneide und/oder Spitze kann das Lanzettenelement optional weitere Elemente umfassen, beispielsweise mindestens ein Element zum Sammeln und/oder Transportieren einer Probe, beispielsweise eine Kapillare und/oder einen Kapillarspalt, und/oder mindestens ein Testelement zum Nachweis mindestens eines Analyten in einer Körperflüssigkeit, beispielsweise eine Testchemie. Auf diese Weise können beispielsweise so genannte Microsampler realisiert werden, welche einen Probenaufnahmevorgang mit einem Probenanalysevorgang verknüpfen.

Das analytische Band ist eingerichtet, um in einem analytischen Testgerät mit mindestens einer Probenaufnahmefunktion verwendet zu werden. Unter einem analytischen Testgerät ist dabei ein Gerät zu verstehen, welches eingesetzt werden kann im Rahmen eines qualitativen und/oder quantitativen Nachweises mindestens eines Analyten in einer Körperflüssigkeit. Dabei kann der eigentliche Nachweis des Analyten unmittelbar durch das analytische Testgerät selbst durchgeführt werden, oder das analytische Testgerät kann lediglich als Hilfsgerät für das eigentliche Nachweisgerät fungieren, beispielsweise als reine Stechhilfe. Auch integrierte Testgeräte mit Stechfunktion und Nachweisfunktion sind möglich. Das analytische Testgerät weist dabei mindestens eine Probenaufnahmefunktion auf. Unter einer Probenaufnahmefunktion ist dabei eine Funktion zu verstehen, mittels derer eine Probe der Körperflüssigkeit generierbar ist, insbesondere eine Lanzettenfunktion. Zusätzlich kann das analytische Testgerät weitere Funktionen aufweisen, beispielsweise eine Analysefunktion zum Nachweis des mindestens einen Analyten in der Körperflüssigkeit. Alternativ kann diese Analysefunktion jedoch auch von einem separaten analytischen Gerät verwendet werden. Das analytische Testgerät kann insbesondere als Handgerät ausgestaltet sein.

Unter einem analytischen Band ist dementsprechend ein zumindest näherungsweise kontinuierlich ausgestaltetes, bandförmiges Element zu verstehen, welches in einer Bandrichtung, also entlang der Längserstreckung des analytischen Bands, eine Mehrzahl analytischer Hilfsmittel umfasst, beispielsweise mindestens 5, vorzugsweise mindestens 10 oder sogar mindestens 20, insbesondere auch mindestens 100 oder mindestens 200 analytische Hilfsmittel. Diese analytischen Hilfsmittel umfassen Lanzettenelemente. Beispielsweise kann das analytische Band ausschließlich Lanzettenelemente als analytische Hilfsmittel umfassen, also als Lanzettenband ausgestaltet sein. Optional können jedoch, wie oben dargestellt, auch andere Arten analytischer Hilfsmittel vorgesehen sein, beispielsweise Testfelder. Darüber hinaus kann das analytische Band beispielsweise Markierungen, Transportelemente (wie beispielsweise Ösen oder Ähnliches) oder andere Elemente umfassen.

Bei dem Verfahren wird mindestens ein Trägerband in einem kontinuierlichen Prozess bereitgestellt. Unter einem kontinuierlichen Prozess ist ein Prozess zu verstehen, bei welchem, im Gegensatz zu Batch-to-Batch-Prozessen, nacheinander und ohne Unterbrechung des Trägerbands gefertigt wird. Insbesondere kann es sich bei diesem kontinuierlichen Prozess um einen Rolle-zu-Rolle-Prozess handeln und/oder der kontinuierliche Prozess kann mindestens einen derartigen Rolle-zu-Rolle-Prozess umfassen. Beispielsweise kann das Trägerband von einer Ausgangsspule bereitgestellt werden und nach Aufbringen der Lanzettenelemente auf eine Produktrolle aufgewickelt werden.

Bei dem Verfahren wird an mindestens einer Applikationsstelle eine Mehrzahl von Lanzettenelementen nacheinander unmittelbar auf das Trägerband aufgebracht. Dabei können auch mehrere Applikationsstellen vorgesehen sein. Beispielsweise kann jede Applikationsstelle eine Lanzettenaufbringstation enthalten, beispielsweise mit einer Stanzstation, bei welcher die Lanzettenelemente beispielsweise unmittelbar aus einem Lanzettenband und/oder einem Vorprodukt herausgenommen, beispielsweise ausgestanzt, werden. Bei der Stanzstation kann ein Ausstanzen der Lanzetten und vorzugsweise auch gleichzeitig ein Positionieren der Lanzetten stattfinden. Beispielsweise kann ein vorgeätztes Metallband bereitgestellt werden, aus welchem die Lanzetten ausgestanzt werden. Das Metallband kann auch Positionierungsmarkierungen umfassen, wie beispielsweise Löcher, welche für die Positionierung des Metallbandes und/oder der Lanzetten relativ zum Trägerband und/oder relativ zu einer Vorrichtung genutzt werden können. Auch eine andere Ausgestaltung der Applikationsstelle ist jedoch grundsätzlich möglich, beispielsweise eine Bereitstellung bereits vereinzelter Lanzetten. Vereinzelte Lanzetten können beispielsweise mittels Saugern, Greifern, Vakuumwalzen oder ähnlichen Applikationselementen auf das Trägerband übertragen werden. Es können dabei ein einzelnes oder auch mehrere Lanzettenelemente gleichzeitig auf das Trägerband übertragen werden, letzteres beispielsweise mit bereits zueinander ausgerichteten Lanzettenelementen.

Unter einem Aufbringen nacheinander ist dabei ein Aufbringen zu verstehen, bei welchem in einer Laufrichtung des Trägerbandes die Lanzettenelemente an unterschiedlichen Stellen des Trägerbands aufgebracht werden, beispielsweise äquidistant in Laufrichtung. Auch ein nicht-äquidistanter Abstand ist jedoch grundsätzlich möglich. Weiterhin können grundsätzlich auch mehrere Lanzettenelemente parallel an derselben Stelle des Lanzettenbandes aufgebracht werden.

Im Unterschied zum oben beschriebenen Stand der Technik, bei welchem die Lanzettenelemente mittels einer Etiketten-Technologie auf das Trägerband appliziert werden, wird bei dem erfindungsgemäßen Verfahren also eine unmittelbare Aufbringung der Lanzettenelemente auf das Trägerband vorgeschlagen. Dies bedeutet, dass zwischen den Lanzettenelementen und dem Trägerband keine diskontinuierlichen Zwischenelemente, wie beispielsweise Trägeretiketten, vorgesehen sind, welche lateral, also in einer Ebene des Trägerbands oder zumindest in einer Längserstreckungsrichtung des Trägerbands, über die Ausdehnung der Lanzettenelemente hinausragen. Auch eine Verwendung zusätzlicher Verbindungselemente, über welche die Lanzettenelemente flexibel mit dem Trägerband verbunden werden, wie beispielsweise in WO 2008/043565 A2, erfolgt nicht. Die Lanzettenelemente liegen also unmittelbar auf dem Trägerband auf. Dies schließt jedoch nicht aus, dass das Trägerband mit einer kontinuierlichen Klebeschicht versehen ist, welche die Haftung der Lanzettenelemente auf dem Trägerband gewährleistet. Allgemein ist es im Rahmen der vorliegenden Erfindung bevorzugt, wenn ein Kleber oder eine Klebeschicht verwendet werden, der bzw. biokompatibel ausgestaltet ist. Alternativ oder zusätzlich können auch die Lanzettenelemente an ihrer dem Trägerband zuweisenden Seite mit einer entsprechenden Klebeschicht versehen sein, wobei die Klebeschicht sich jedoch lateral vorzugsweise nicht über die laterale Ausdehnung der Lanzettenelemente hinaus erstreckt. Die Klebeschicht weist in diesem Fall also maximal dieselbe Grundfläche wie die Lanzettenelemente auf oder eine kleinere Grundfläche.

Besonders bevorzugt ist dabei, wenn das analytische Band derart ausgestaltet ist, dass dieses im Wesentlichen eine konstante Dicke aufweist. Unter einer im Wesentlichen konstanten Dicke ist dabei im Rahmen der vorliegenden Erfindung eine konstante Dicke zu verstehen, wobei jedoch Abweichungen von bis zu 20%, vorzugsweise von nicht mehr als 10% und besonders bevorzugt von nicht mehr als 5% noch tolerierbar sind. Diese konstante Dicke wird dabei vorzugsweise lediglich an den Lanzettenelementen durch die Dicke der Lanzettenelemente erhöht. Dementsprechend kann das analytische Band beispielsweise zweistufig ausgestaltet sein, mit einer ersten Dicke, nämlich der konstanten Dicke des analytischen Bandes selbst, und einer zweiten Dicke im Bereich der Lanzettenelemente. Dickenänderungen an weiteren Elementen, wie beispielsweise Etiketten-Kanten, wie aus dem Stand der Technik bekannt, treten in dieser Ausgestaltung nicht auf.

Das direkte Aufbringen der Lanzetten auf das Trägerband weist gegenüber den bekannten Etikettier-Verfahren eine Vielzahl von Vorteilen auf. So kann das analytische Band derart ausgestaltet werden, dass dieses lediglich die Kanten der Lanzettenelemente als Dickenänderung enthält. Gleichzeitig lässt sich jedoch bei diesem Verfahren, wie auch bei anderen Verfahren eine hohe Sterilität der analytischen Bänder gewährleisten. Das Verfahren lässt sich großtechnisch als Rolle-zu-Rolle-Verfahren ausgestalten, so dass auch eine großtechnische Umsetzung des Verfahrens mit hohen Durchsatzraten gewährleistet werden kann.

Ein wesentlicher Vorteil des direkten Aufbringens der Lanzettenelemente auf das Trägerband liegt insbesondere in dem Erkennungsprinzip der beförderten Lanzettenelemente durch das analytische Testgerät, beispielsweise eine Transporteinrichtung des analytischen Testgeräts. Beispielsweise kann diese Transporteinrichtung einen Greifer und/oder einen Sensor, beispielsweise einen mit einem Greifer kombinierten Sensor, zum Erkennen eines Lanzettenelements und zum entsprechenden Positionieren des Lanzettenelements umfassen. Diesbezüglich kann beispielsweise auf die bereits oben zitierte WO 2008/138443 A1 verwiesen werden, welche auch im Rahmen der vorliegenden Erfindung einsetzbar ist. Auch auf die WO 2009/037341 A1 und das dort beschriebenen analytische Testgerät kann verwiesen werden. Insbesondere in derartigen analytischen Testgeräten machen sich die vorgeschlagenen analytischen Bänder beziehungsweise analytische Bänder, welche nach dem vorgeschlagenen Verfahren hergestellt sind, besonders positiv bemerkbar. Beispielsweise lassen sich die Lanzettenelemente dort auf einfache und zuverlässige Weise durch den Greifer erkennen. Die Erkennung durch den Greifer funktioniert dabei vorzugsweise zumindest teilweise über die Dickenänderung des Lanzettenelements gegenüber dem analytischen Band außerhalb der Testelemente. Im Gegensatz zu bekannten analytischen Bändern und Herstellungsverfahren unter Verwendung von Etiketten, bei denen der Greifer jede Dickenänderung als neue Lanzette erkennt, obwohl es sich hierbei lediglich um eine Faltkante oder ein unterschiedlich großes Etikett handeln kann, erfolgt bei den vorgeschlagenen analytischen Bändern eine zuverlässige und sichere Erkennung. Die vorgeschlagenen analytischen Bänder erfüllen auch höchste Präzisionsanforderungen, welche auch in hohem Durchsatz beispielsweise mit einem Rolle-zu-Rolle-Verfahren, gewährleistet werden können.

Das vorgeschlagene Verfahren sowie entsprechende Ausgestaltungen des analytischen Bandes lassen sich auf verschiedene Weisen vorteilhaft weiterbilden. So ist es, wie oben dargestellt, besonders bevorzugt, wenn die Lanzettenelemente Flachlanzetten umfassen oder sogar gänzlich als Flachlanzetten ausgestaltet sind. Auf diese Weise kann insbesondere die oben beschriebene sprunghafte Dickenänderung gewährleistet werden, welche für die Erkennung der Lanzettenelemente, insbesondere eine automatische Erkennung besonders vorteilhaft ist. Diese Flachlanzetten können mit einer Flachseite auf das Trägerband aufgebracht werden.

Das Verfahren kann vorzugsweise derart durchgeführt werden, dass mindestens ein biokompatibler Kleber und/oder mindestens eine biokompatible Klebeschicht verwendet werden. Dieser biokompatible Kleber beziehungsweise diese biokompatible Kleberschicht kann beispielsweise ein Kleber beziehungsweise eine Klebeschicht sein, welche mit den Lanzettenelementen, insbesondere einer Schneide und/oder einer Spitze der Lanzettenelemente, in Kontakt kommt. Dieser biokompatible Kleber und/oder diese biokompatible Klebeschicht kann beispielsweise eingesetzt werden, um die Lanzettenelemente mit dem Trägerband zu verbinden und/oder um eine kontinuierliche Abdeckung auf die Lanzetten aufzubringen. Auch an anderen Stellen können ein derartiger biokompatibler Kleber und/oder eine biokompatible Klebeschicht alternativ oder zusätzlich eingesetzt werden.

In einer weiteren vorteilhaften Ausgestaltung des Verfahrens kann zusätzlich mindestens eine kontinuierliche Abdeckung auf die Lanzetten aufgebracht werden. Eine derartige kontinuierliche Abdeckung, im Gegensatz zu einzelnen Taschen oder einzelnen Schutzelementen für die Lanzettenelemente, wie sie aus dem Stand der Technik bekannt sind, wird also vorzugsweise in dem vorgeschlagenen Verfahren eine kontinuierliche Abdeckung verwendet. Eine derartige kontinuierliche Abdeckung kann insbesondere mit einem Rolle-zu-Rolle-Verfahren aufgebracht werden. Beim Gebrauch der Lanzettenelemente kann diese kontinuierliche Abdeckung beispielsweise durch die Lanzettenelemente durchstoßen, durchstochen oder durchschnitten werden. Alternativ kann eine derartige kontinuierliche Abdeckung bereits, wie unten noch näher dargestellt wird, auch Teil des Trägerbands selbst sein. Grundsätzlich wäre, alternative oder zusätzlich zu einer kontinuierlichen Abdeckung, jedoch auch eine diskontinuierliche Abdeckung denkbar, beispielsweise mittels einzelner Abdeck-Patches, welche einzeln aufgebracht werden. Dies ist jedoch aufgrund der oben genannten Nachteile weniger bevorzugt.

Unter einem Aufbringen auf die Lanzettenelemente wird dabei ein Aufbringen auf eine dem Trägerband gegenüberliegende Seite der Lanzettenelemente verstanden. Eine derartige Abdeckung gewährleistet insbesondere eine Sterilhaltung der der Lanzettenelemente.

Zur Erzeugung einer derartigen kontinuierlichen Abdeckung können verschieden Verfahren eingesetzt werden, welche einzeln oder auch in Kombination anwendbar sind. So kann beispielsweise zur Herstellung der kontinuierlichen Abdeckung, wie oben dargestellt, das Trägerband selbst verwendet werden und dieses kann entlang einer Faltkante parallel zu einer Längsrichtung des Trägerbands umgefaltet werden. So kann beispielsweise mindestens eine Kante des Trägerbands nach dem Aufbringen der Lanzettenelemente über die Lanzettenelemente gefaltet werden. Zu diesem Zweck können die Lanzettenelemente beispielsweise asymmetrisch auf das Trägerband aufgebraucht werden, so dass eine ausreichende Kante zur anschließenden Abdeckung zur Verfügung steht. Die umgefaltete Kante kann beispielsweise mit dem nicht-umgefalteten Trägerband stoffschlüssig verbunden werden, insbesondere verklebt und/oder verschweißt werden. Der Vorteil eines Faltverfahrens liegt darin, dass eine Faltkante in aller Regel besonders dicht ausgestaltet ist. Beispielsweise können die Lanzettenelemente derart auf dem Trägerband angeordnet sein, dass Schneiden und/oder Spitzen der Lanzettenelemente der Faltkante zuweisen und durch diese besonders geschützt sind, beispielsweise gegenüber einem Eindringen von Feuchtigkeit und/oder Verschmutzungen. Alternativ oder zusätzlich zur Herstellung der kontinuierlichen Abdeckung mittels eines Faltverfahrens kann zum Herstellen der Abdeckung auch mindestens ein zusätzliches kontinuierliches Abdeckband über die Lanzetten aufgebracht werden. Auch dieses kann wiederum beispielsweise mit dem Trägerband stoffschlüssig verbunden werden, insbesondere verklebt und/oder verschweißt werden. Das Abdeckband kann beispielsweise eine Dicke von 2 bis 10 Mikrometern aufweisen, beispielsweise von 4 Mikrometern bis 8 Mikrometern und besonders bevorzugt von 6 Mikrometern aufweisen.

Das Trägerband kann beispielsweise ein Kunststoffband umfassen. Beispielsweise kann es sich dabei um ein Polyethylen-Terephthalat (PET-Material) und/oder ein Polyethylen-Material handeln. Auch der Einsatz anderer Arten von Kunststoffen und/oder anderer Materialien, beispielsweise Papiermaterialien und/oder Keramikmaterialien, ist grundsätzlich möglich. Alternativ können auch Laminatbänder verwendet werden, also Trägerbänder, welche aus mehreren Materialien und/oder mehreren Schichten zusammengesetzt sind.

Entsprechend kann auch das optionale Abdeckband ausgestaltet sein. Auch dieses kann beispielsweise aus einem Kunststoffmaterial und/oder einem anderen der genannten Materialien für das Trägerband hergestellt werden. Auch eine Ausgestaltung, bei welcher das Abdeckband eine andere Materialauswahl aufweist als das Trägerband ist jedoch grundsätzlich möglich.

Wie oben dargestellt, können die Lanzettenelemente unmittelbar auf dem Trägerelement fixiert werden, insbesondere durch eine stoffschlüssige Verbindung, vorzugsweise eine Klebung. Diese Klebung kann auf verschiedene Weise ausgestaltet sein, beispielsweise in Form einer kontinuierlichen Klebeschicht auf dem Trägerband und/oder einer Klebeschicht auf einer dem Trägerband zuweisenden Seite der Lanzettenelemente, welche lateral vorzugsweise nicht über die Ausdehnung der Lanzettenelemente herausragt. Dementsprechend kann beispielsweise das Trägerband mindestens eine Grundschicht und mindestens eine Klebeschicht aufweisen. Die Grundschicht kann beispielsweise aus einem oder mehreren der oben genannten Materialien zusammengesetzt sein. Die zusätzliche Klebeschicht kann beispielsweise als dünne Schicht auf diese Grundschicht aufgebracht sein. Das Aufbringen der Klebeschicht kann auch Teil des beschriebenen Verfahrens sein oder es kann bereits ein Trägerband bereitgestellt werden, welches diese Klebeschicht aufweist. Die Grundschicht kann beispielsweise eine Dicke von 2 bis 20 Mikrometern aufweisen, insbesondere von 5 bis 15 Mikrometern und besonders bevorzugt von 6 bis 12 Mikrometern, beispielsweise 6 Mikrometer oder 12 Mikrometer. Die Klebeschicht kann beispielsweise eine Dicke von 5 bis 30 Mikrometern aufweisen, insbesondere von 10 bis 20 Mikrometern. Die Klebeschicht kann dann nicht nur zur Fixierung der Lanzettenelemente genutzt werden, sondern auch zur Fixierung der kontinuierlichen Abdeckung nach Aufbringen derselben. Wird ein zusätzliches kontinuierliches Abdeckband über die Lanzetten aufgebracht so kann alternativ oder zusätzlich jedoch auch dieses kontinuierliche Abdeckband mindestens eine Klebeschicht aufweisen.

Weiterhin kann das Verfahren derart ausgestaltet werden, dass zusätzlich auf einer den Lanzettenelementen abgewandten Seite des Trägerbands mindestens ein Stabilisierungsband, vorzugsweise ein kontinuierliches Stabilisierungsband, aufgebracht wird. Dieses Aufbringen des Stabilisierungsbands kann vorzugsweise nach dem Aufbringen der Lanzettenelemente erfolgen. Das Stabilisierungsband kann beispielsweise wiederum eines oder mehrere der oben bezüglich des Trägerbands beschriebenen Materialien aufweisen. Auch eine andere Materialauswahl bezüglich des Stabilisierungsbands ist grundsätzlich möglich. Das Stabilisierungsband kann beispielsweise eine Dicke zwischen 2 und 10 Mikrometern aufweisen, insbesondere von 6 Mikrometern. Zwar ist grundsätzlich die Verwendung eines zusätzlichen Stabilisierungsbands in vielen Fällen zu vermeiden, da dieses zumindest einen zusätzlichen Verfahrensschritt erfordert und grundsätzlich vermieden werden kann, wenn ein ausreichend stabiles Trägerband verwendet wird. Wird jedoch das oben beschriebene Verfahren verwendet, bei welchem zur Erzeugung der kontinuierlichen Abdeckung mindestens eine Kante des Trägerbands nach dem Aufbringen der Lanzettenelemente über die Lanzettenelemente gefaltet wird, so kann die Verwendung eines dünnen Trägerbands geboten sein. Insbesondere in diesen Fällen, jedoch auch in anderen Fällen, kann dann die Verwendung eines Stabilisierungsbands auf der gegenüberliegenden Seite vorteilhaft sein.

Wie oben mehrfach dargestellt, wird neben dem vorgeschlagenen Verfahren weiterhin ein analytisches Band vorgeschlagen. Dieses kann insbesondere herstellbar sein nach einem erfindungsgemäßen Verfahren gemäß einer oder mehreren der oben beschriebenen Ausführungsvarianten. Dementsprechend kann für mögliche Ausgestaltungen des analytischen Bands auf die obige Beschreibung des Verfahrens verwiesen werden. Das analytische Band ist eingerichtet, um in einem analytischen Testgerät mit mindestens einer Probenaufnahmefunktion verwendet zu werden. Das analytische Band weist mindestens ein kontinuierlich ausgebildetes Trägerband auf, wobei eine Mehrzahl von Lanzettenelementen nacheinander unmittelbar auf das Trägerband aufgebracht ist. Bezüglich der Vorteile des analytischen Bands kann auf die obige Beschreibung des Verfahrens verwiesen werden.

Weiterhin wird ein analytisches Testgerät vorgeschlagen, welches mindestens eine Probenaufnahmefunktion im Sinne der obigen Beschreibung umfasst. Das analytische Testgerät kann insbesondere als Handgerät ausgestaltet sein. Das analytische Testgerät umfasst ein analytisches Band gemäß der obigen Beschreibung, also in einer oder mehreren der oben beschriebenen Ausgestaltungen. Weiterhin weist das analytische Testgerät mindestens eine Transporteinrichtung auf, welche eingerichtet ist, um jeweils ein Lanzettenelement in eine Anwendungsposition zu bringen. Beispielsweise kann diese Anwendungsposition eine Position sein, in welcher mittels des jeweiligen Lanzettenelements eine Probenaufnahmebewegung, beispielsweise eine Stechbewegung, optional verbunden mit einem Sammelvorgang zum Aufnehmen von Körperflüssigkeit, durchführbar ist. Zu diesem Zweck kann das analytische Testgerät beispielsweise einen entsprechenden Aktuationsmechanismus umfassen, um die Stechbewegung durchzuführen. Die Transporteinrichtung kann insbesondere eine Rollentransporteinrichtung umfassen, beispielsweise mit einem Gutwickel und einem Schlechtwickel, wobei auf dem Gutwickel unbenutzte Bereiche des analytischen Bands aufgewickelt sind und auf dem Schlechtwickel Bereiche mit bereits benutzten Lanzettenelementen. Dementsprechend kann das analytische Testgerät beispielsweise eingerichtet sein, um jedes Lanzettenelement ausschließlich einfach zu verwenden. Die Transporteinrichtung kann insbesondere mindestens einen Greifer umfassen, welcher jeweils mindestens ein Lanzettenelement greifen kann. Die Transporteinrichtung ist allgemein eingerichtet, um ein Vorhandensein eines Lanzettenelements anhand einer Dickenänderung des analytischen Bands zu erkennen. Aufgrund der Verwendung des erfindungsgemäßen analytischen Bands beziehungsweise eine erfindungsgemäß hergestellten analytischen Bands lassen sich die oben dargestellten Vorteile realisieren, und der Transportmechanismus kann seine Funktion besonders störungsunempfindlich und zuverlässig erfüllen. Für eine Ausgestaltung des analytischen Testgeräts kann dabei exemplarisch, mit Ausnahme des analytischen Bandes, auf den oben zitierten Stand der Technik verwiesen werden, insbesondere auf die WO 2008/138443 A1. Das dort beschriebene analytische Testgerät kann jedoch auch modifiziert werden, um insbesondere die beschriebene Dickenänderung zu detektieren, beispielsweise mittels eines separaten Detektors beziehungsweise Sensors und/oder mittels des genannten Greifers. Auch andere Ausgestaltungen des analytischen Testgeräts sind jedoch grundsätzlich möglich.

Weiterhin wird eine Vorrichtung zur Herstellung eines analytischen Bands vorgeschlagen. Die Vorrichtung ist eingerichtet, um ein Verfahren zur Herstellung eines analytischen Bands in einer oder mehreren der oben beschriebenen Verfahrensvarianten durchzuführen. Dementsprechend kann weit gehend auf die obige Beschreibung verwiesen werden, und die Vorrichtung kann jeweils eine oder mehrere Teil-Vorrichtungen zur Durchführung der beschriebenen Verfahrensschritte umfassen. Beispielsweise kann die Vorrichtung mindestens eine Bereitstellvorrichtung umfassen, um das mindestens eine Trägerband in einem kontinuierlichen Prozess bereitzustellen. Beispielsweise kann diese Bereitstellvorrichtung eine oder mehrere Rollen umfassen. Weiterhin kann er die Vorrichtung mindestens eine Applikationsvorrichtung, beispielsweise eine Lanzettenaufbringstation umfassen, um an mindestens einer Applikationsstelle die Mehrzahl von Lanzettenelementen nacheinander unmittelbar auf das Trägerband aufzubringen. Weiterhin können zur Durchführung der optionalen Verfahrensschritte weitere Teil-Vorrichtungen vorgesehen sein, beispielsweise mindestens eine Falteinheit und/oder mindestens eine Bereitstellvorrichtung für mindestens eine kontinuierliche Abdeckung, insbesondere ein Abdeckband und/oder mindestens eine Bereitstellvorrichtung für mindestens eine rückseitige Abdeckung, insbesondere ein Stabilisierungsband. Ausführungsbeispiele einer derartigen Vorrichtung sind der folgenden Beschreibung zu entnehmen.

### Kurze Beschreibung der Figuren

Weitere Einzelheiten und Merkmale ergeben sich aus der nachfolgenden Beschreibung, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche beziehungsweise hinsichtlich ihrer Funktionen einander entsprechende Elemente.

Im Einzelnen zeigen:
- Figuren 1A bis 1D: verschiedene Ansichten eines ersten Ausführungsbeispiels eines erfindungsgemäßen Verfahrens mit einer kontinuierlichen Abdeckung in Form einer Faltung einer Kante des Trägerbands;
- Figuren 2A bis 2C: verschiedene Ansichten eines zweiten Ausführungsbeispiels eines erfindungsgemäßen Verfahrens, bei welchem zur Erzeugung einer kontinuierlichen Abdeckung zusätzlich ein kontinuierliches Abdeckband aufgebracht wird;
- Figuren 3A bis 3C: verschiedene Ansichten eines dritten Ausführungsbeispiels eines erfindungsgemäßen Verfahrens, bei welchem zusätzlich ein rückseitiges Stabilisierungsband aufgebracht wird; und
- Figur 4: ein Ausführungsbeispiel eines Metallbands zur Bereitstellung einer Mehrzahl von Lanzettenelementen.

### Ausführungsbeispiele

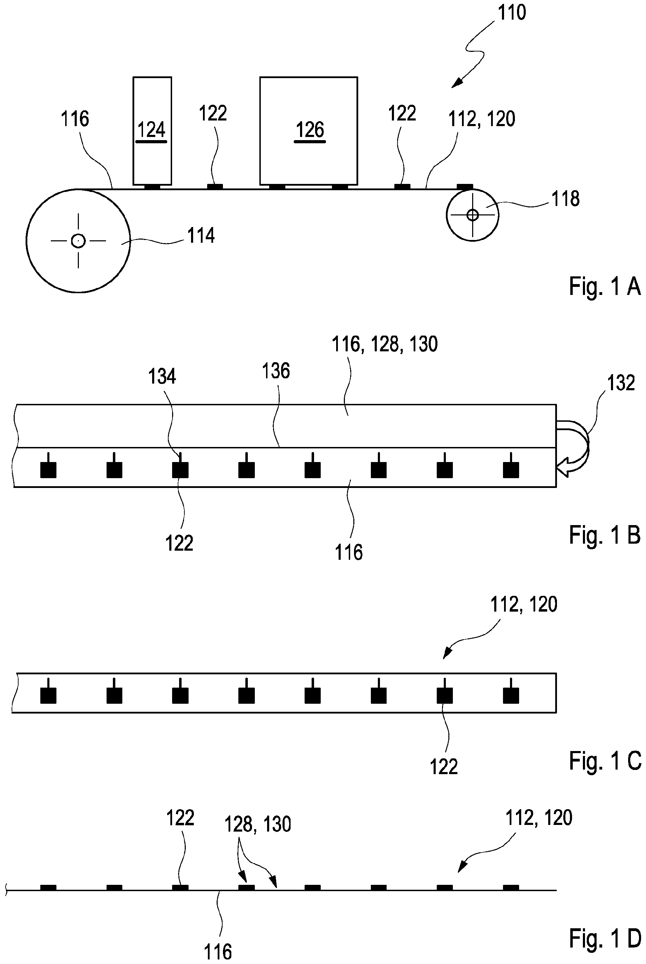

In den Figuren 1A bis 1D ist ein erstes Ausführungsbeispiel eines Verfahrens in verschiedenen Ansichten gezeigt. Dabei zeigt Figur 1A eine Seitenansicht einer Vorrichtung zur Durchführung des Verfahrens, Figur 1B zeigt ein Zwischenprodukt vor einem Umfalten einer Kante eines Trägerbands und die Figuren 1C und 1D zeigen eine Draufsicht beziehungsweise eine Seitenansicht eines fertigen analytischen Bands.

In Figur 1A ist also eine Vorrichtung 110 zur Herstellung eines analytischen Bands 112 schematisch dargestellt, anhand derer das erste Ausführungsbeispiel des Verfahrens erläutert werden soll. Die Vorrichtung umfasst eine erste Rolle 114 zur Bereitstellung eines Trägerbands 116. Nach Herstellung des analytischen Bands 112 wird dann das fertige analytische Band 112 und/oder ein Vorprodukt desselben, welches noch weiteren Verfahrensschritten unterworfen werden kann, auf eine zweite Rolle 118 aufgewickelt.

Das Trägerband kann in diesem Ausführungsbeispiel beispielsweise eine Kunststoff-Folie mit einer Dicke von 6 Mikrometern sein, auf welches eine Klebeschicht von beispielsweise 10 bis 20 Mikrometern aufgebracht ist oder aufgebracht wird. Dementsprechend kann als Trägerband 116 beispielsweise ein herkömmlicher Klebestreifen verwendet werden. Für die in den Figuren nicht gesondert bezeichnete optionale Klebeschicht wird dabei vorzugsweise ein biokompatibler Klebstoff verwendet. Auf diese Weise kann die Klebeschicht gefahrlos mit den Lanzettenelementen 122, insbesondere Lanzettenspitzen 134 dieser Lanzettenelemente 122, in Berührung kommen, welche später bei der Anwendung kurzfristig in den Körper eindringen.

Das analytische Band 112 ist in diesem Ausführungsbeispiel als Lanzettenband 120 ausgestaltet. Dementsprechend werden eine Vielzahl von Lanzettenelementen 122 an einer Lanzettenaufbringstation 124 an einer Applikationsstelle nacheinander unmittelbar auf das Trägerband 116 aufgebracht.

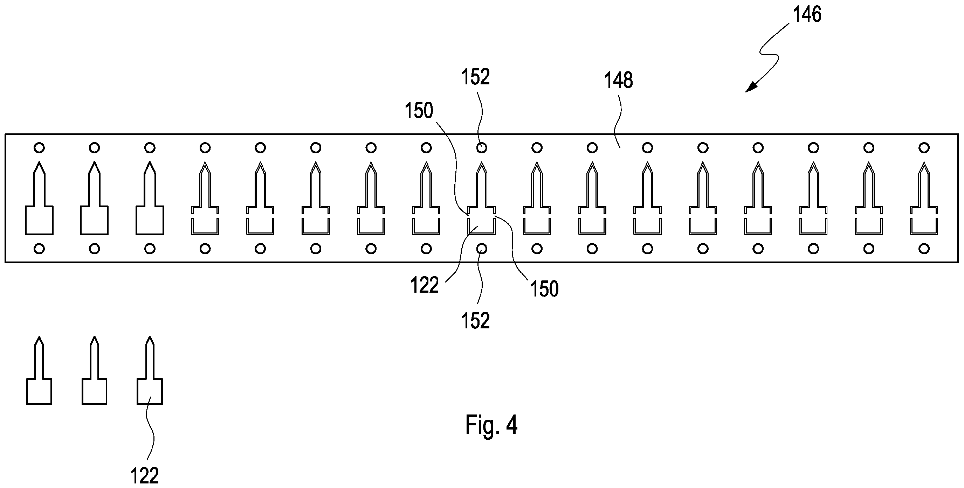

In Figur 4 ist ein Ausführungsbeispiel eines Metallbands 146 dargestellt, mittels dessen eine Mehrzahl von Lanzettenelemente in 122 an der Lanzettenaufbringstation 124 bereitgestellt werden kann, beispielsweise in einem kontinuierlichen Rollenprozess. Das Metallband 146 kann ein Metallträgerband 148 umfassen, aus welchem die Lanzettenelemente 122 in ihren Konturen vorgeätzt sind, so dass diese beispielsweise lediglich noch durch dünne Stege 150 mit dem restlichen Metallträgerband 148 verbunden sind. Diese Stege 150 können beispielsweise an der Lanzettenaufbringstation 124 durchtrennt werden, beispielsweise durch einen Stanzprozess und/oder einen Schneideprozess. Die Lanzettenaufbringstation 124 kann zu diesem Zweck entsprechende Stanz- und/oder Schneideelemente umfassen und/oder andere Elemente, um die Stege 150 zu durchtrennen. Im linken unteren Teil der Figur 4 sind drei bereits herausgetrennte Lanzettenelemente 122 dargestellt.

Weiterhin kann das Metallband 146, insbesondere das Metallträgerband 148, eine oder mehrere Positionierungshilfen umfassen, welche beispielsweise eine Positionierung des Metallbands 146 relativ zur Lanzettenaufbringstation 124 und/oder zum Trägerband 116 und/oder zu einem sonstigen Teil einer Vorrichtung zur Herstellung eines analytischen Bands 112, beispielsweise der in Figur 1A gezeigten Vorrichtung, ermöglichen. Im dargestellten Ausführungsbeispiel in Figur 4 sind als Beispiele derartiger Positionierungshilfen Löcher 152 vorgesehen. Diese Löcher können beispielsweise beidseitig der Lanzettenelemente 122 in dem Metallträgerband 148 vorgesehen sein, so dass beispielsweise jedem Lanzettenelement 122 genau ein Loch 152 oder genau zwei derartiger Löcher 152 zugeordnet sind. Die Löcher können beispielsweise wiederum durch ein Ätzverfahren erzeugt werden, beispielsweise durch dasselbe Ätzverfahren, mittels dessen auch die Lanzettenelemente 122 aus dem Metallträgerband 148 herausgeätzt werden.

Die Vorrichtung 110 zur Herstellung des analytischen Bands 112 in Figur 1A oder auch in anderen Ausführungsbeispielen kann eine Vorrichtung zur Bereitstellung des Metallbands 146 umfassen, beispielsweise wiederum eine Vorrichtung mit einer oder mehreren Rollen, beispielsweise einem Gutwickel und/oder einem Schlechtwickel. Mit dieser Vorrichtung kann an der Lanzettenaufbringstation 124 eine Bereitstellung der Lanzettenelemente 122 erfolgen. Es wird jedoch darauf hingewiesen, dass auch eine andere Art der Bereitstellung erfolgen kann, beispielsweise eine Bereitstellung mittels einer anderen Ausgestaltung eines Bandes und/oder eine Bereitstellung einzelner Lanzettenelemente 122, beispielsweise als Schüttgut und/oder als einzeln verpackte Lanzettenelemente 122. Die Bereitstellung gemäß Figur 4 ist jedoch technisch besonders einfach zu realisieren, und es kann ein hoher Reinheitsgrad gewährleistet werden.

Wie aus den Figuren 1B bis 1C hervorgeht, wird in der Vorrichtung 110 gemäß Figur 1A vorzugsweise in dieser Ausgestaltung des Verfahrens ein Trägerband 116 verwendet, welches breiter ist als die Endbreite des fertigen analytischen Bandes 112, beispielsweise ein Trägerband 116, welches zumindes näherungsweise doppelt so breit ist wie die Endbreite des fertigen analytischen Bands 112. Wie oben dargestellt, ist dieses Trägerband 116 vorzugsweise auf der der Lanzettenaufbringstation 124 zuweisenden Seite mit einer Klebeschicht versehen. Nach dem Aufbringen der Lanzettenelemente 122 durchläuft das Trägerband 116 eine Falteinheit 126. Dabei wird, wie in Figuren 1B und 1C erkennbar ist, eine Kante 128 als kontinuierliche Abdeckung 130 verwendet und über die Lanzettenelemente 122 gefaltet. Der Faltvorgang ist in Figur 1B symbolisch mit der Bezugsziffer 132 bezeichnet. Aufgrund der Klebeschicht haftet die umgefaltete Kante 128 auf dem nicht umgefalteten Bereich des Trägerbands 116 sowie auf den Lanzettenelementen 122, so dass diese verschlossen und damit versiegelt werden. Vorzugsweise weisen Lanzettenspitzen 134 dabei einer Faltkante 136 zu, da diese Faltkante 136 besonders dicht ist und praktisch keine Gefahr für eine Sterilschutzgewährleistung in sich birgt. Auch eine andere Ausrichtung ist jedoch grundsätzlich möglich. In anderen Fällen muss jedoch in der Regel eine Mindestdichtigkeit der auf diese Weise erzeugten kontinuierlichen Abdeckung 130 nachgewiesen werden, da geklebte Kanten in der Regel Poren enthalten können, welche den Sterilschutz in der Regel nicht mehr gewährleisten. Nach Durchlaufen der Falteinheit 126 wird dann das derart erzeugte analytische Band 112 auf die zweite Rolle 118 aufgewickelt.

Ein Vorteil des auf diese Weise sowie mittels der anderen Ausführungsbeispiele hergestellten analytischen Bandes 112 liegt darin, dass dieses eine konstante, durchgehende gleiche Dicke aufweist, welche nur durch die Lanzettenelemente 122 unterbrochen wird. Zusätzliche Dickenvariationen, beispielsweise in Form von Stufen, an Etiketten oder ähnlichen Elementen, treten in der Regel nicht auf. Auf diese Weise kann besonders zuverlässig und sicher ein Weitertaktmechanismus in einem analytischen Gerät eingesetzt werden wie beispielsweise dem in WO 2008/138443 A1 beschriebenen Testgerät.

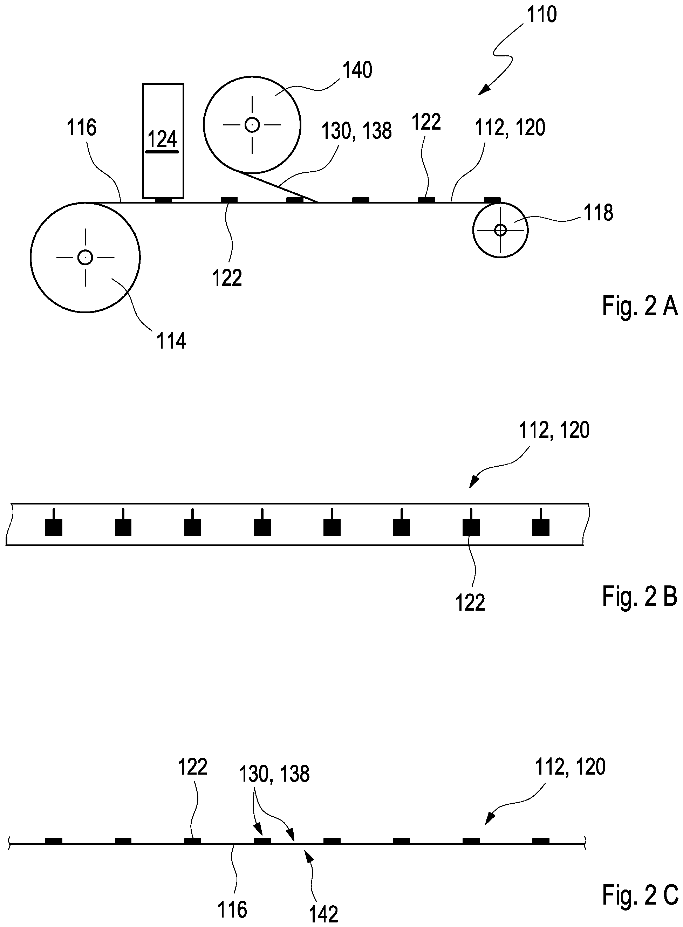

In den Figuren 2A bis 2C ist ein zweites Ausführungsbeispiel eines Verfahrens zur Herstellung eines analytischen Bands 112, wiederum in Form eines Lanzettenbands 120, dargestellt. Dabei zeigt wiederum Figur 2A, in analoger Darstellung zur Figur 1A eine Vorrichtung 110 zur Durchführung des Verfahrens in stark schematisierter Ansicht. Die Figuren 2B und 2C zeigen wiederum, in analoger Darstellung zu den Figuren 1C und 1D, ein fertiges analytisches Band 112 in Draufsicht beziehungsweise in Seitenansicht.

Wiederum umfasst die Vorrichtung 110 eine erste Rolle 114 zur kontinuierlichen Bereitstellung eines Trägerbands 116, beispielsweise wiederum eines Trägerbands 116 mit einer Klebeschicht. Weiterhin umfasst die Vorrichtung 110, welche wiederum zur Durchführung eines Rolle-zu-Rolle-Verfahrens ausgestaltet sein kann, eine zweite Rolle 118 zum Aufwickeln des fertigen analytischen Bands 112 und/oder eines Zwischenprodukts desselben, welches anschließend noch weiter verarbeitet werden kann. Wiederum ist zudem eine Lanzettenaufbringstation 124 zum Aufbringen von Lanzettenelementen 122 vorgesehen, welche beispielsweise wiederum, wie auch in den Figuren 1A bis 1D, als Stanzstation ausgestaltet sein kann und/oder eine derartige Stanzstation umfassen kann.

Wiederum wird bei dem Ausführungsbeispiel in den Figuren 2A bis 2C eine kontinuierliche Abdeckung 130 über den Lanzettenelementen 122 aufgebracht. Im Unterschied zu dem in den Figuren 1A bis 1D beschriebenen Faltverfahren wird hier die kontinuierliche Abdeckung jedoch in Form eines zusätzlichen kontinuierlichen Abdeckbands 138 aufgebracht. Dieses zusätzliche kontinuierliche Abdeckband kann beispielsweise wiederum mittels eines kontinuierlichen Prozesses, beispielsweise eines Rolle-zu-Rolle-Prozesses, von einer dritten Rolle 140 bereitgestellt werden.

Das Trägerband 116 kann beispielsweise wiederum eine Kunststoff-Folie umfassen. Das Trägerband kann beispielsweise eine Dicke von 12 Mikrometern aufweisen und optional mit einer Klebeschicht versehen sein, beispielsweise einer Klebeschicht mit einer Dicke von 10 bis 20 Mikrometern, wobei die Klebeschicht vorzugsweise wiederum kontinuierlich auf das Trägerband 116 aufgebracht ist oder aufgebracht wird. Das Abdeckband 138 kann beispielsweise in Form einer Abdeckfolie ausgestaltet sein, beispielsweise wiederum einer Kunststoff-Folie. Das Abdeckband 138 kann beispielsweise eine Dicke von 6 Mikrometern aufweisen. Dieses dient der Bereitstellung eines Sterilschutzes. Das Abdeckband 138 muss nicht notwendigerweise mit einer Klebeschicht versehen sein, kann jedoch zusätzlich optional eine derartige Klebeschicht umfassen. Vorzugsweise sind wiederum sämtliche verwendete Klebeschichten im dargestellten Ausführungsbeispiel biokompatibel ausgestaltet.

Wie in Figur 2A dargestellt ist die Aufbringung des zusätzlichen kontinuierlichen Abdeckbands 138 der Aufbringung der Lanzettenelemente 122 nachgeschaltet. Weiterhin können, wie auch in dem vorhergehenden Ausführungsbeispiel, zusätzliche, in den Figuren nicht dargestellte Verfahrensschritte vorgesehen sein. Das fertige oder halbfertige analytische Band 112 wird dann auf die zweite Rolle 118 aufgewickelt.

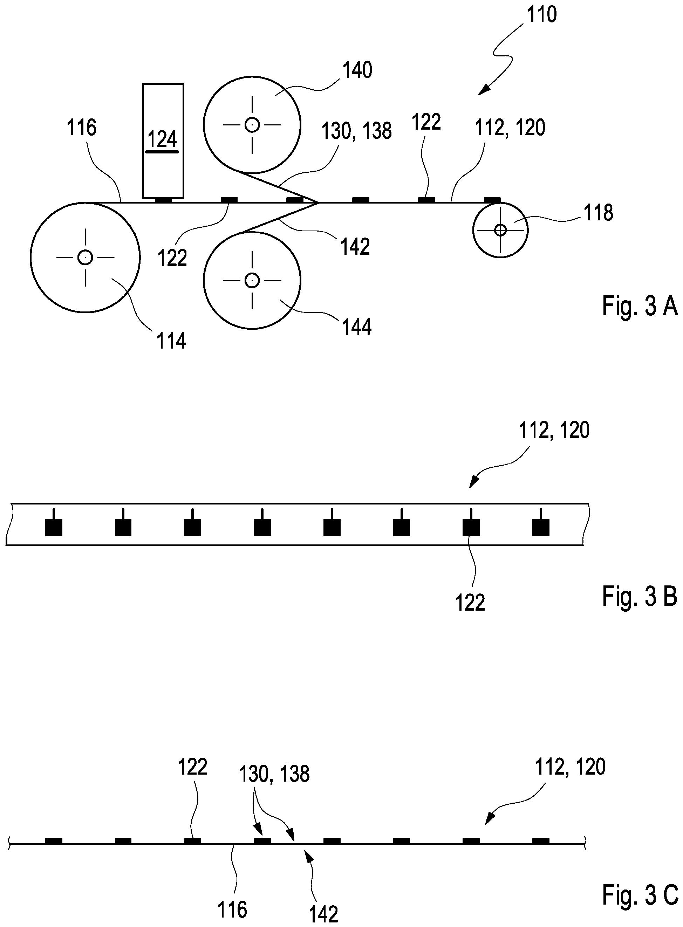

In den Figuren 3A bis 3C ist, in analoger Darstellung zu den Figuren 2A bis 2C ein drittes Ausführungsbeispiel eines Verfahrens gezeigt. Dabei zeigt Figur 2A wiederum eine Vorrichtung 110 zur Durchführung des Verfahrens, wohingegen die Figuren 3B und 3C fertige oder halbfertige Verfahrensprodukte in Form eines analytischen Bands 112, welches vorzugsweise wiederum als Lanzettenband 120 ausgestaltet ist, zeigen.

Das Verfahren verläuft grundsätzlich zunächst analog zu dem Verfahren in den Figuren 2A bis 2C, so dass weitgehend auf die obige Beschreibung verwiesen werden kann. Wiederum wird mit Hilfe einer dritten Rolle 140 eine kontinuierliche Abdeckung 130 in Form separaten kontinuierlichen Abdeckbands 138 auf das Trägerband 116 mit den aufgebrachten Lanzettenelementen 122 aufgebracht. Zusätzlich wird jedoch, beispielsweise nach dem Aufbringen der Lanzettenelemente 122, auf der den Lanzettenelementen 122 abgewandten Seite des Trägerbands 116 mindestens ein Stabilisierungsband 142 auf das Trägerband 116 aufgebracht. Dies kann beispielsweise wiederum mittels eines kontinuierlichen Prozesses erfolgen, so dass ein kontinuierliches Trägerband 142 in einem Rolle-zu-Rolle-Prozess bereitgestellt werden kann, beispielsweise von einer vierten Rolle 144. Die Aufbringung des Stabilisierungsbands 142 kann dabei grundsätzlich an einer beliebigen Stelle erfolgen, beispielsweise, wie in Figur 3A dargestellt, gleichzeitig mit der Aufbringung des Abdeckbands 138. Auch eine andere Ausgestaltung ist jedoch möglich, beispielsweise eine frühere oder eine spätere Aufbringung.

Bei dieser Verfahrensvariante in den Figuren 3A bis 3C kann beispielsweise ein dünneres Trägerband 116 als in Ausführungsbeispielen 2A und 2C eingesetzt werden. Beispielsweise kann ein Trägerband mit einer Dicke von 6 Mikrometern und optional einer Klebeschicht von beispielsweise 10 bis 20 Mikrometern verwendet werden. Diese Klebeschicht kann dabei vorzugsweise beidseitig auf dem Trägerband 116 aufgebracht sein. Alternativ oder zusätzlich kann jedoch auch das Stabilisierungsband 142 eine entsprechende Klebeschicht umfassen, um das Stabilisierungsband 142 auf der Rückseite des Trägerbands 116 zu fixieren. Das Abdeckband kann beispielsweise wiederum eine Dicke von 6 Mikrometern aufweisen, so dass ein Sterilschutz gewährleistet ist. Das Stabilisierungsband kann beispielsweise eine Dicke von 6 Mikrometern aufweisen.

Nach Herstellung eines derartigen analytischen Bands 112 kann dann das derart hergestellt Produkt oder Halbfertigprodukt wiederum, wie in Figur 3A dargestellt, auf der zweiten Rolle 118 aufgewickelt werden. Dieses kann dann gegebenenfalls noch weiteren Verfahrensschritten unterworfen werden. In den Figuren 3B und 3C sind fertige Verfahrensprodukte dargestellt, welche jedoch gegebenenfalls ebenfalls noch weiteren Verfahrensschritten unterworfen werden können.

Die dargestellten Ausführungsbeispiele zeigen dabei Beispiele, in welchen reine Lanzettenbänder 120 hergestellt werden. Alternativ können jedoch zusätzlich weitere analytische Elemente aufgebracht werden, beispielsweise Testfelder oder Ähnliches.

### Bezugszeichenliste

- 110: Vorrichtung zur Herstellung eines analytischen Bands
- 112: analytisches Band
- 114: erste Rolle
- 116: Trägerband
- 118: zweite Rolle
- 120: Lanzettenband
- 122: Lanzettenelemente
- 124: Lanzettenaufbringstation
- 126: Falteinheit
- 128: Kante
- 130: kontinuierliche Abdeckung
- 132: Falten
- 134: Lanzettenspitzen
- 136: Faltkante
- 138: Abdeckband
- 140: dritte Rolle
- 142: Stabilisierungsband
- 144: vierte Rolle
- 146: Metallband
- 148: Metallträgerband
- 150: Stege
- 152: Löcher

## Patentansprüche

1. Verfahren zur Herstellung eines analytischen Bandes (112), wobei das analytische Band (112) eingerichtet ist, um in einem analytischen Testgerät mit mindestens einer Probennahmefunktion verwendet zu werden, wobei bei dem Verfahren mindestens ein Trägerband (116) in einem kontinuierlichen Prozess bereitgestellt wird, wobei an mindestens einer Applikationsstelle eine Mehrzahl von Lanzettenelementen (122) nacheinander unmittelbar auf das Trägerband (116) aufgebracht wird, wobei die Lanzettenelemente (122) runde Nadeln oder Flachlanzetten sind und durch eine stoffschlüssige Verbindung unmittelbar auf dem Trägerband (116) fixiert werden, wobei das analytische Band (112) derart ausgestaltet wird, dass dieses im Wesentlichen eine konstante Dicke aufweist, wobei die konstante Dicke lediglich an den Lanzettenelementen (122) durch die Dicke der Lanzettenelemente (122) erhöht ist.

2. Verfahren nach dem vorhergehenden Anspruch, wobei die Lanzettenelemente (122) Flachlanzetten sind, wobei die Flachlanzetten mit einer Flachseite auf das Trägerband (116) aufgebracht werden.

3. Verfahren nach einem der vorhergehenden Ansprüche, wobei mindestens ein biokompatibler Kleber und/oder mindestens eine biokompatible Klebeschicht verwendet werden.

4. Verfahren nach einem der vorhergehenden Ansprüche, wobei das Trägerband (116) mindestens eine Grundschicht und mindestens eine Klebeschicht aufweist.

5. Verfahren nach dem vorhergehenden Anspruch, wobei die Grundschicht eine Dicke von 2 bis 20 Mikrometern aufweist, insbesondere von 5 bis 15 Mikrometern und besonders bevorzugt von 6 Mikrometern bis 12 Mikrometern.

6. Verfahren nach einem der beiden vorhergehenden Ansprüche, wobei die Klebeschicht eine Dicke von 5 bis 30 Mikrometern aufweist, insbesondere von 10 bis 20 Mikrometern.

7. Verfahren nach einem der vorhergehenden Ansprüche, wobei zusätzlich mindestens eine kontinuierliche Abdeckung (130) auf die Lanzettenelemente (122) aufgebracht wird.

8. Verfahren nach dem vorhergehenden Anspruch, wobei zur Herstellung der kontinuierlichen Abdeckung (130) mindestens eine Kante (128) des Trägerbands (116) nach dem Aufbringen der Lanzettenelemente (122) über die Lanzettenelemente (122) gefaltet wird.

9. Verfahren nach dem vorhergehenden Anspruch, wobei die umgefaltete Kante (128) mit dem nicht-umgefalteten Trägerband (116) stoffschlüssig verbunden wird, insbesondere verklebt und/oder verschweißt wird.

10. Verfahren nach einem der drei vorhergehenden Ansprüche, wobei zum Herstellen der kontinuierlichen Abdeckung (130) mindestens ein zusätzliches kontinuierliches Abdeckband (138) über die Lanzettenelemente (122) aufgebracht wird.

11. Verfahren nach dem vorhergehenden Anspruch, wobei das Abdeckband (138) eine Dicke von 2 bis 10 Mikrometern, insbesondere eine Dicke von 4 Mikrometern bis 8 Mikrometern und besonders bevorzugt von 6 Mikrometern aufweist.

12. Verfahren nach einem der vorhergehenden Ansprüche, wobei zusätzlich auf einer den Lanzettenelementen (122) abgewandten Seite des Trägerbands (116) mindestens ein Stabilisierungsband (142) aufgebracht wird.

13. Verfahren nach dem vorhergehenden Anspruch, wobei das Stabilisierungsband (142) eine Dicke zwischen 2 und 10 Mikrometern aufweist, insbesondere von 6 Mikrometern.

14. Analytisches Band (112), wobei das analytische Band (112) eingerichtet ist, um in einem analytischen Testgerät mit mindestens einer Probennahmefunktion verwendet zu werden, wobei das analytische Band (112) mindestens ein kontinuierlich ausgebildetes Trägerband (116) aufweist, wobei eine Mehrzahl von Lanzettenelementen (122) nacheinander unmittelbar auf das Trägerband (116) aufgebracht ist, wobei die Lanzettenelemente (122) runde Nadeln oder Flachlanzetten sind und durch eine stoffschlüssige Verbindung unmittelbar auf dem Trägerband (116) fixiert sind, wobei das analytische Band (112) derart ausgestaltet ist, dass dieses im Wesentlichen eine konstante Dicke aufweist, wobei die konstante Dicke lediglich an den Lanzettenelementen (122) durch die Dicke der Lanzettenelemente (122) erhöht ist.

15. Analytisches Testgerät, aufweisend mindestens eine Probennahmefunktion, weiterhin umfassend ein analytisches Band (112) nach dem vorhergehenden Anspruch, wobei das analytische Testgerät mindestens eine Transporteinrichtung aufweist, insbesondere eine Transporteinrichtung mit mindestens einem Greifer, wobei die Transporteinrichtung eingerichtet ist, um jeweils ein Lanzettenelement (122) in eine Anwendungsposition zu bringen, wobei die Transporteinrichtung eingerichtet ist, um ein Vorhandensein eines Lanzettenelements (122) anhand einer Dickenänderung des analytischen Bands (112) zu erkennen.

16. Vorrichtung (110) zur Herstellung eines analytischen Bands (112) nach Anspruch 14 , wobei die Vorrichtung (110) eingerichtet ist, um ein Verfahren nach einem der vorhergehenden, ein Verfahren betreffenden Ansprüche durchzuführen, wobei die Vorrichtung (110) mindestens eine Bereitstellvorrichtung (114, 118) umfasst, um das mindestens eine Trägerband (116) in einem kontinuierlichen Prozess bereitzustellen und wobei die Vorrichtung (110) weiterhin mindestens eine Applikationsvorrichtung (124) umfasst, um an mindestens einer Applikationsstelle die Mehrzahl von Lanzettenelementen (122) nacheinander durch eine stoffschlüssige Verbindung unmittelbar auf das Trägerband (116) aufzubringen, wobei die Lanzettenelemente (122) runde Nadeln oder Flachlanzetten sind, wobei die Applikationsvorrichtung Lanzettenaufbringstation (124) ist.

## Claims

1. Method for producing an analytic tape (112), wherein the analytic tape (112) is configured to be used in an analytic test device having at least one sampling function, wherein at least one carrier tape (116) is provided in a continuous process within the method, wherein a plurality of lancet elements (122) are directly applied to the carrier tape (116) in succession at at least one application point, wherein the lancet elements (122) are round needles or flat lancets and directly affixed to the carrier tape (116) by an integral connection, wherein the analytic tape (112) is configured in such a way that the latter has a substantially constant thickness, wherein the constant thickness is only increased at the lancet elements (122) by the thickness of the lancet elements (122).

2. Method according to the preceding claim, wherein the lancet elements (122) are flat lancets, wherein the flat lancets are applied to the carrier tape (116) with a flat side.

3. Method according to either of the preceding claims, wherein use is made of at least one biocompatible adhesive and/or at least one biocompatible adhesive layer.

4. Method according to any one of the preceding claims, wherein the carrier tape (116) has at least one base layer and at least one adhesive layer.

5. Method according to the preceding claim, wherein the base layer has a thickness of 2 to 20 micrometres, in particular of 5 to 15 micrometres and particularly preferably of 6 micrometres to 12 micrometres.

6. Method according to either one of the two preceding claims, wherein the adhesive layer has a thickness of 5 to 30 micrometres, in particular of 10 to 20 micrometres.

7. Method according to any one of the preceding claims, wherein additionally at least one continuous cover (130) is applied to the lancet elements (122).

8. Method according to the preceding claim, wherein at least one edge (128) of the carrier tape (116) is folded over the lancet elements (122) following the application of the lancet elements (122) for the purposes of producing the continuous cover (130).

9. Method according to the preceding claim, wherein the folding edge (128) is integrally connected, in particular adhesively bonded and/or welded, to the non-folded carrier tape (116).

10. Method according to any one of the three preceding claims, wherein at least one additional continuous covering tape (138) is applied over the lancet elements (122) for the purposes of producing the continuous cover (130).

11. Method according to the preceding claim, wherein the covering tape (138) has a thickness of 2 to 10 micrometres, in particular a thickness of 4 micrometres to 8 micrometres and particularly preferably of 6 micrometres.

12. Method according to any one of the preceding claims, wherein additionally at least one stabilizing tape (142) is applied to the side of the carrier tape (116) facing away from the lancet elements (122).

13. Method according to the preceding claim, wherein the stabilizing tape (142) has a thickness of between 2 and 10 micrometres, in particular of 6 micrometres.

14. Analytic tape (112), wherein the analytic tape (112) is configured to be used in an analytic test device having at least one sampling function, wherein the analytic tape (112) has at least one continuously embodied carrier tape (116), wherein a plurality of lancet elements (122) are directly applied to the carrier tape (116) in succession, wherein the lancet elements (122) are round needles or flat lancets and directly affixed to the carrier tape (116) by an integral connection, wherein the analytic tape (112) is configured in such a way that the latter has a substantially constant thickness, wherein the constant thickness is only increased at the lancet elements (122) by the thickness of the lancet elements (122).

15. Analytic test device, having at least one sampling function, furthermore comprising an analytic tape (112) according to the preceding claim, wherein the analytic test device has at least one transportation device, in particular a transportation device with at least one gripper, wherein the transportation device is configured to bring respectively one lancet element (122) into an application position, wherein the transportation device is configured to identify the presence of a lancet element (122) on the basis of a change in thickness and the analytic tape (112).

16. Apparatus (110) for producing an analytic tape (112) according to Claim 14, wherein the apparatus (110) is configured to carry out a method according to any one of the preceding claims that relates to a method, wherein the apparatus (110) comprises at least one provision apparatus (114, 118) for providing the at least one carrier tape (116) in a continuous process and wherein the apparatus (110) furthermore comprises at least one application apparatus (124) for successively applying a plurality of lancet elements (122) directly to the carrier tape (116) at at least one application point by way of an integral connection, wherein the lancet elements (122) are round needles or flat lancets, wherein the application apparatus is a lancet application station (124).

## Revendications

1. Procédé de fabrication d'une bande analytique (112), la bande analytique (112) étant conçue pour être utilisée dans un testeur analytique ayant au moins une fonction de prélèvement d'échantillon, lors du procédé au moins une bande porteuse (116) étant fournie en un processus continu, une pluralité d'éléments formant lancette (122) étant appliqués les uns après les autres directement sur la bande porteuse (116) en au moins un point d'application, les éléments formant lancette (122) étant des aiguilles rondes ou des lancettes plates et étant calés directement sur la bande porteuse (116) par une liaison par fusion de matières, la bande analytique (112) étant configurée de telle sorte que celle-ci présente une épaisseur sensiblement constante, l'épaisseur constante étant uniquement accrue au niveau des éléments formant lancette (122) par l'épaisseur des éléments formant lancette (122).

2. Procédé selon la revendication précédente, les éléments formant lancette (122) étant des lancettes plates, les lancettes plates étant appliquées sur la bande porteuse (116) par un côté plat.

3. Procédé selon l'une des revendications précédentes, au moins un adhésif biocompatible et/ou au moins une couche adhésive biocompatible étant utilisés.

4. Procédé selon l'une des revendications précédentes, la bande porteuse (116) possédant au moins une couche de base et au moins une couche adhésive.

5. Procédé selon la revendication précédente, la couche de base présentant une épaisseur de 2 à 20 micromètres, notamment de 5 à 15 micromètres et notamment de préférence de 6 micromètres à 12 micromètres.

6. Procédé selon l'une des deux revendications précédentes, la couche adhésive présentant une épaisseur de 5 à 30 micromètres, notamment de 10 à 20 micromètres.

7. Procédé selon l'une des revendications précédentes, au moins un recouvrement continu (130) étant en plus appliqué sur les éléments formant lancette (122).

8. Procédé selon la revendication précédente, en vue de produire le recouvrement continu (130), au moins un bord (128) de la bande porteuse (116) étant plié au-dessus des éléments formant lancette (122) après l'application des éléments formant lancette (122).

9. Procédé selon la revendication précédente, le bord (128) replié étant relié par fusion de matières à la bande porteuse (116) non repliée, notamment collé et/ou soudé.

10. Procédé selon l'une des trois revendications précédentes, au moins une bande de recouvrement continue (138) supplémentaire étant appliquée sur les éléments formant lancette (122) en vue de produire le recouvrement continu (130).

11. Procédé selon la revendication précédente, la bande de recouvrement (138) présentant une épaisseur de 2 à 10 micromètres, notamment une épaisseur de 4 micromètres à 8 micromètres et notamment de préférence de 6 micromètres.

12. Procédé selon l'une des revendications précédentes, au moins une bande de stabilisation (142) étant en plus appliquée sur un côté de la bande porteuse (116) à l'opposé des éléments formant lancette (122).

13. Procédé selon la revendication précédente, la bande de stabilisation (142) présentant une épaisseur entre 2 et 10 micromètres, notamment de 6 micromètres.

14. Bande analytique (112), la bande analytique (112) étant conçue pour être utilisée dans un testeur analytique ayant au moins une fonction de prélèvement d'échantillon, la bande analytique (112) possédant au moins une bande porteuse (116) de configuration continue, une pluralité d'éléments formant lancette (122) étant appliqués les uns après les autres directement sur la bande porteuse (116) en au moins un point d'application, les éléments formant lancette (122) étant des aiguilles rondes ou des lancettes plates et étant calés directement sur la bande porteuse (116) par une liaison par fusion de matières, la bande analytique (112) étant configurée de telle sorte que celle-ci présente une épaisseur sensiblement constante, l'épaisseur constante étant uniquement accrue au niveau des éléments formant lancette (122) par l'épaisseur des éléments formant lancette (122).

15. Testeur analytique, possédant au moins une fonction de prélèvement d'échantillon, comprenant en outre une bande analytique (112) selon la revendication précédente, le testeur analytique possédant au moins un dispositif de transport, notamment un dispositif de transport pourvu d'au moins un préhenseur, le dispositif de transport étant conçu pour amener respectivement un élément formant lancette (122) dans une position d'utilisation, le dispositif de transport étant conçu pour reconnaître une présence d'un élément formant lancette (122) à l'aide d'un changement d'épaisseur de la bande analytique (112) .

16. Arrangement (110) de fabrication d'une bande analytique (112) selon la revendication 14, l'arrangement (110) étant conçu pour mettre en œuvre un procédé selon l'une des revendications précédentes concernant un procédé, l'arrangement (110) comportant au moins un arrangement de fourniture (114, 118) pour fournir l'au moins une bande porteuse (116) en un processus continu, l'arrangement (110) comportant en outre un arrangement d'application (124) pour appliquer la pluralité d'éléments formant lancette (122) les uns après les autres directement sur la bande porteuse (116) en au moins un point d'application par une liaison par fusion de matières, les éléments formant lancette (122) étant des aiguilles rondes ou des lancettes plates, l'arrangement d'application étant une station d'application de lancettes (124).
